# EUROPEAN PATENT APPLICATION

(11) **EP 2 449 995 A1**
(43) Date of publication of application: **09.05.2012**
(21) Application number: 10794263.3
(22) Date of filing: 05.07.2010
(51) Int. Cl.: A61B 19/00, A61B 17/28, A61B 1/00

(54) **FORCEPS SUPPORT DEVICE**

(30) Priority: 03.07.2009 JP 2009159309
(71) Applicant: Kyushu University, National University Corporation, Fukuoka-shi, Fukuoka 812-8581 (JP)
(72) Inventor: HASHIZUME, Makoto, Fukuoka-shi Fukuoka 812-8581 (JP); KENMOTSU, Hajime, Fukuoka-shi Fukuoka 812-8581 (JP); YUGE, Kazuo, Narashino-shi Chiba 275-0001 (JP)
(74) Representative: Nicholls, Michael John
(86) International application number: PCT/JP2010/061370
(87) International publication number: WO 2011/002095

(57) **Abstract**

A forceps support device 1 for supporting forceps 60 used for endoscopic treatment, comprising a frame 2, a pair of forceps support portions 11 provided at both ends of the frame 2, forceps support means 20 provided to each of the forceps support portions 11 to support the forceps 60 pivotably in the horizontal direction and the vertical direction, and drive means for pivoting the forceps support means 20 in the horizontal direction and the vertical direction. The forceps support means 20 each include a first arm 21 joined to a forceps support portion 11 pivotably in the horizontal direction, a second arm 27 joined to the forceps support portion 11 pivotably in the vertical direction, and a forceps support member 35 that spans the first arm 21 and the second arm 27, is pivotable in the horizontal direction while following the first arm 21, and pivotable in the vertical direction while following the second arm 27. The forceps 60 are supported by the forceps support members 35.

## Description

### Technical Field

The present invention relates to a forceps support device, and more particularly relates to a forceps support device that is effective at supporting forceps used in treatment of disorders of the digestive tract and so forth, along with an endoscope, in endoscopic treatment and the like performed on the digestive tract and so forth in the abdominal cavity of a human patient.

### Background Art

In recent years, when treatment is performed for a disorder of the digestive tract or the like in the abdominal cavity of a human patient, an endoscope is inserted through the mouth, nose, etc., into the body, and the disorder of the digestive tract or the like is treated using forceps attached to the endoscope, which minimizes the invasiveness of the procedure on the patient.

An endoscopic surgical robot such as that discussed in Patent Literature 1, for example, has been used in such endoscopic treatments. This endoscopic surgical robot includes an endoscope, a pair of forceps arms, a head support portion for supporting the endoscope and the pair of forceps arms, and forceps provided to the distal ends of the forceps arms.

Each of the forceps consists of a first sleeve attached to the head support portion, a second sleeve linked via a first flexible tube to the first sleeve, a pair of forceps attached openably and closeably to the distal end of a second flexible tube, three oscillating levers pivotably attached around the second sleeve, wires for opening and closing the pair of forceps, and wires for pivoting the oscillating levers.

With the endoscopic surgical robot constituted as above, the wires are manipulated to pivot one of the oscillating levers, and as a result the first flexible tube follows its oscillating lever and bends at the first sleeve, and the pair of forceps follow the first flexible tube and pivot in the same direction via the second sleeve and the second flexible tube. When a wire is manipulated to pivot the other oscillating lever in a state in which the first flexible tube has been bent, the first flexible tube follows its oscillating lever and bends further in that direction, and the pair of forceps follow the bending of the first flexible tube and rotate in that direction via the second sleeve and the second flexible tube.

The endoscopic surgical robot having the above-mentioned functions is inserted through the mouth, nose, etc., into the body, the oscillating levers are pivoted by manipulation of the wires, thereby pivoting or rotating the pair of forceps, the forceps are positioned at a position corresponding to the disorder in the digestive tract or the like, and the forceps are opened and closed by manipulating the wires at this position, allowing a specific treatment to be performed on the disorder in the digestive tract or the like.

### Citation List

### Patent Literature

Patent Document 1: JP2004-180781A

### Summary of Invention

### Technical Problem

However, with an endoscopic surgical robot constituted as above, since the forceps are positioned at a position corresponding to the disorder in the digestive tract or the like by manipulating the wires to pivot the oscillating levers and bend the first flexible tube, so that the forceps pivot or rotate by following the first flexible tube, the positioning precision of the forceps ends up being dependent on the bending responsiveness of the first flexible tube, and the forceps cannot be accurately positioned at the desired position.

Also, when the forceps are placed at a disorder in the digestive tract or the like, and then the position of each forceps in the forward and backward direction is adjusted, either the wires have to be manipulated to re-pivot the oscillating levers and bend the first flexible tube, or the entire endoscopic surgical robot has to be moved forward and backward to adjust the position of the pair of forceps in the forward and backward direction, making it impossible to fine-tune the position of the forceps accurately in the forward and backward direction.

Furthermore, since the forceps are able to open and close in the same plane, it is difficult to perform any forward and backward crossing that may be necessary to the treatment.

The present invention was conceived in light of these problems encountered in the past, and it is an object thereof to provide a forceps support device with which a pair of forceps can be accurately positioned at the desired position, fine adjustment can be accurately performed at that position, and the device can be used for disorders in many different forms.

### Solution to Problem

To solve the above problems, the present invention employs the following means.
Specifically, the invention according to claim 1 is a forceps support device for supporting forceps used for endoscopic treatment, comprising a frame, a pair of forceps support portions provided at both ends of the frame, forceps support means provided to each of the forceps support portions to support the forceps pivotably in the horizontal direction and the vertical direction, and drive means for pivoting the forceps support means in the horizontal direction and the vertical direction.

With the forceps support device of the present invention, the forceps are supported pivotably in the horizontal and vertical directions by the forceps support means, and the forceps support means is driven by the drive means, the result being that the forceps follow the forceps support means as they pivot in the horizontal and vertical directions, and the forceps can be positioned accurately at the desired position by adjusting the pivot angle in the horizontal and vertical directions with the forceps support means. Also, since the forceps can each be pivoted independently in the horizontal and vertical directions, the angle of the pair of forceps can be adjusted as desired, so that the forceps can be used for disorders in many different forms, and can be applied to a wide variety of disorders.

The invention according to claim 2 is the forceps support device according to claim 1, wherein the forceps support means each includes a first arm joined to each of the forceps support portions pivotably in the horizontal direction, a second arm joined to each of the forceps support portions pivotably in the vertical direction, and a forceps support member that spans the first arm and the second arm, is pivotable in the horizontal direction while following the first arm, and pivotable in the vertical direction while following the second arm, and wherein the forceps are supported by the forceps support members.

With the forceps support device of the present invention, the first arm is pivoted horizontally, which causes the forceps support members and the forceps to follow the first arm and pivot in the same direction, and the second arm is pivoted vertically, which causes the forceps support members and the forceps to follow the second arm and pivot in the same direction. Therefore, the pivot angle of the forceps in the horizontal direction can be adjusted with the first arm, and the pivot angle in the vertical direction can be adjusted with the second arm, so the forceps can be positioned accurately at the desired position in the horizontal and vertical directions, and fine tuning of the position can also be performed accurately.

The invention according to claim 3 is the forceps support device according to claim 2, wherein the drive means includes a first wire that is linked at one end to the first arm and can move back and forth in the lengthwise direction of the frame, a second wire that is linked at one end to the second arm and can move back and forth in the lengthwise direction of the frame, and an actuator for moving the first wire and the second wire back and forth.

With the forceps support device of the present invention, the first wire is moved forward or backward by the actuator, the result being that the first arm is pivoted horizontally via the first wire, and the forceps can follow the first arm and pivot in the same direction via the forceps support member. Also, the second wire is moved forward or backward by the actuator, the result being that the second arm is pivoted vertically via the second wire, and the forceps can follow the second arm and pivot in the same direction via the forceps support member.

The invention according to claim 4 is the forceps support device according to claim 2, wherein the forceps support members support the forceps rotatably and slidably.

With the forceps support device of the present invention, the forceps can be rotated and slid, and the forward and backward position and the rotational angle of the forceps can be adjusted. Also, since the forceps support members can support existing forceps, these existing forceps can continue to be used, without having to use new forceps.

The invention according to claim 5 is the forceps support device according to claim 4, wherein the forceps support members includes an outer tube that spans the first arm and the second arm, and an inner tube that is slidably inserted on the inner peripheral side of the outer tube, and bearing portions that rotatably engage the outer tube with the first arm and the second arm are provided at both ends of the outer tube.

With the forceps support device of the present invention, the forceps support members spanning the first arm and the second arm are such that bearings at both ends of an outer tube are rotatably engaged with the first arm and second arm, so when the first arm is pivoted horizontally and the forceps support members and the forceps are pivoted in the same direction following the first arm, the parts of the forceps support members in contact with the second arm rotate via the bearing portions of the outer tube, and when the second arm is pivoted horizontally and the forceps support members and the forceps are pivoted in the same direction following the second arm, the parts of the forceps support members in contact with the first arm rotate via the bearing portions of the outer tube, so that the forceps support members and the first flexible tube can be pivoted smoothly and in the same direction following the pivoting of the first arm in the horizontal direction and the pivoting of the second arm in the vertical direction.

The invention according to claim 6 is the forceps support device according to any of claims 1 to 5, wherein an endoscope support portion is provided to the frame, and an endoscope is supported by the endoscope support portion, via endoscope support means, so as to be able to move back and forth in the lengthwise direction of the frame.

With the forceps support device of the present invention, the endoscope is moved forward or backward in the lengthwise direction of the frame by the endoscope support means, which allows the lens of the endoscope to be moved in the forward and backward direction.

The invention according to claim 7 is the forceps support device according to claim 6, wherein the endoscope support means includes a slide member mounted slidably in the lengthwise direction to the frame, and the endoscope is supported by the slide member.

With the forceps support device of the present invention, the slide member of the endoscope support means is slid in the lengthwise direction of the frame, which allows the endoscope supported by the slide member to be moved in the lengthwise direction of the frame, and allows the lens of the endoscope to be positioned at the desired position.

### Advantageous Effects of Invention

As described above, with the forceps support device of the present invention, a pair of forceps can be positioned accurately at the desired position, and the position of the forceps at the desired location can be fine-tuned very precisely. Also, since the forceps can each be independently pivoted in the horizontal and vertical directions, the angle of each forceps can be adjusted as needed, the forceps can be used for disorders in many different forms, and their applicable range can be expanded.

### Brief Description of Drawings

FIG. 1 is an oblique view of an embodiment of the forceps support device of the present invention, showing the forceps in their open state;
FIG. 2 is a plan view of FIG. 1.
FIG. 3 is a rear view of FIG. 1.
FIG. 4 is a left side view of FIG. 1.
FIG. 5 is a front view of FIG. 1.
FIG. 6 is an oblique view of the forceps in their open state.
FIG. 7 is a plan view of FIG. 6.
FIG. 8 is a rear view of FIG. 6.
FIG. 9 is a left side view of FIG. 6.
FIG. 10 is a front view of FIG. 6.
FIG. 11 is an enlarged view in which the upper plate has been removed from the frame in FIG. 7.
FIG. 12 is a diagram showing the relation between the forceps support portions, the first arm, the second arm, and the outer tube.
FIG. 13 is a diagram showing the relation between the frame, the forceps support portions, and the forceps support means.
FIG. 14 is an enlarged view of the forceps support members.
FIG. 15 is a front view of another embodiment of the forceps support device of the present invention.
FIG. 16 is an enlarged cross section along the A-A line in FIG. 15.
FIG. 17 is an enlarged view of the first stopper portion in FIG. 15.
FIG. 18 is an oblique view of an inner tube.
FIG. 19 is a diagram showing the relation between a wire guide, a wire attachment portion, an outer tube, and an inner tube in FIG. 15.
FIG. 20 is an enlarged lateral cross section of FIG. 19.
FIG. 21 is a view along the arrow B in FIG. 19.

### Description of Embodiments

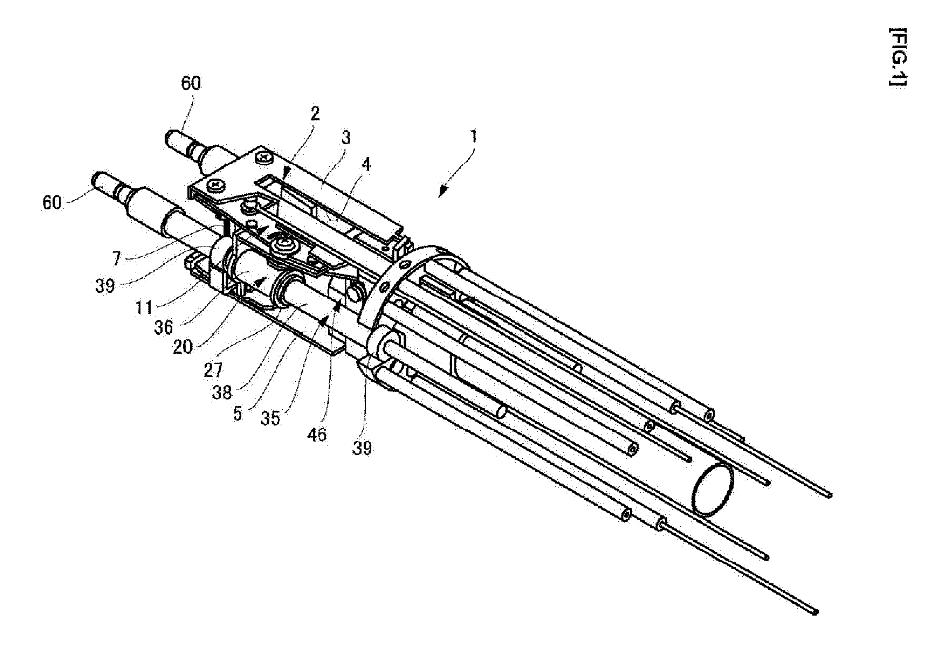

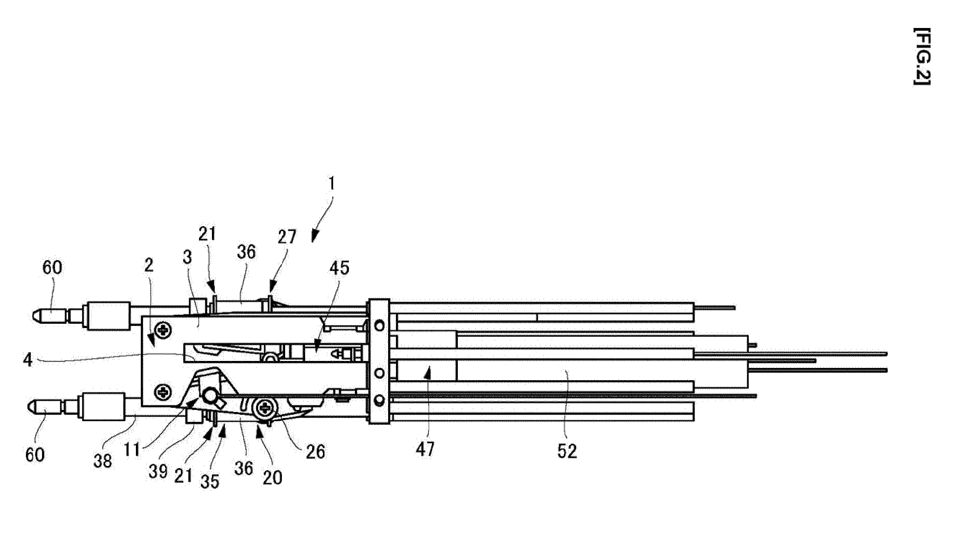

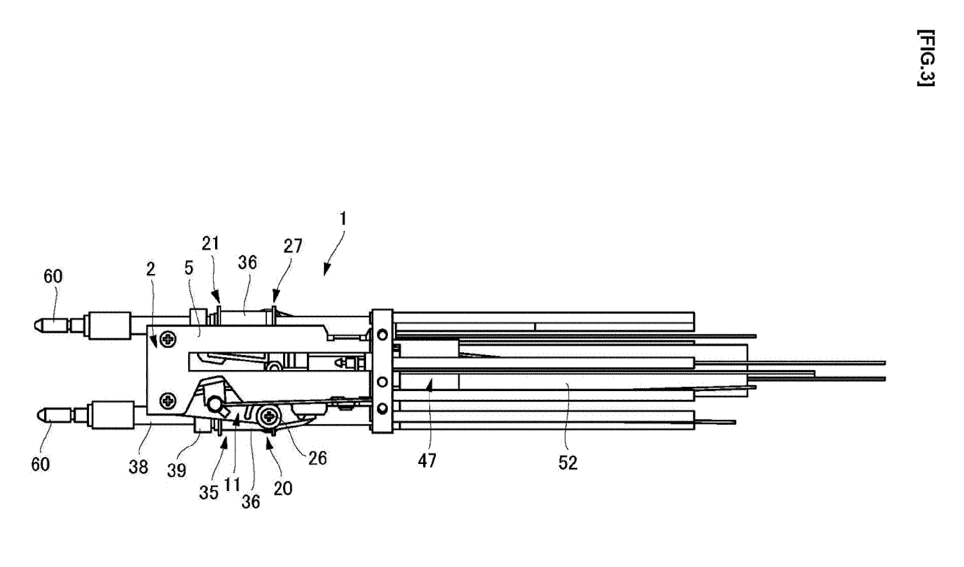

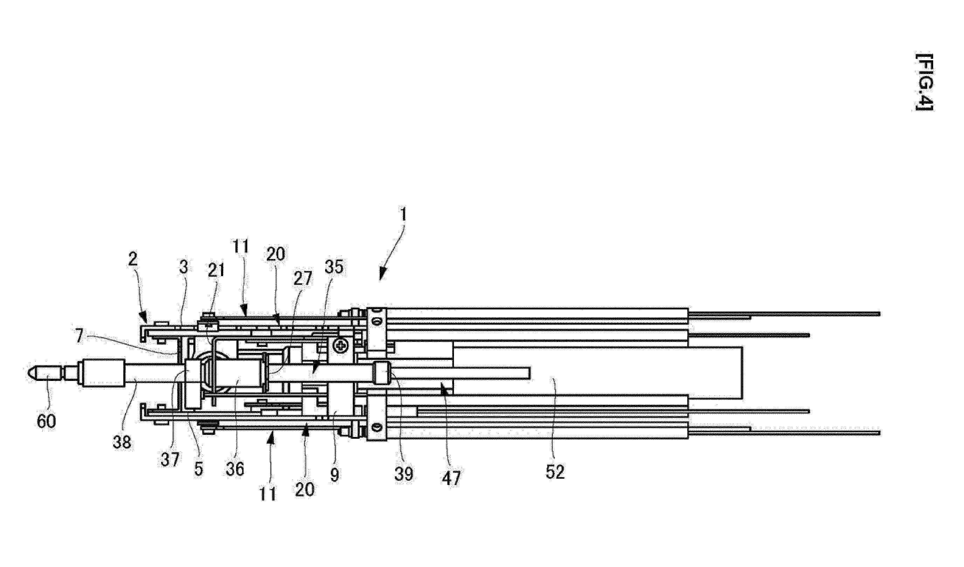

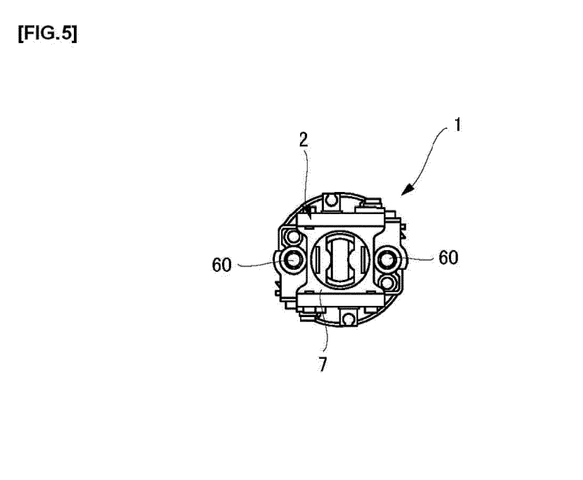

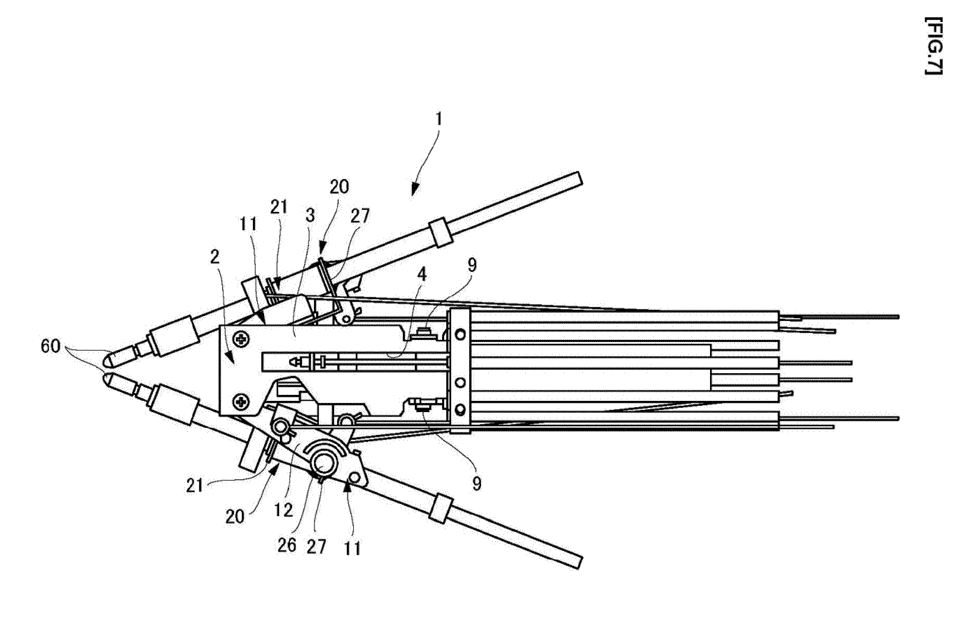

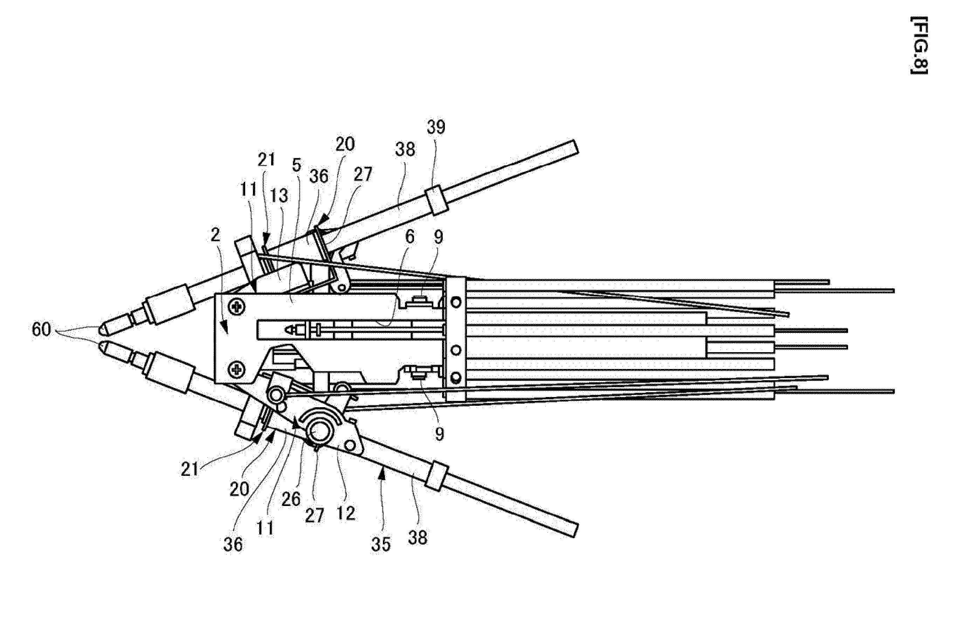

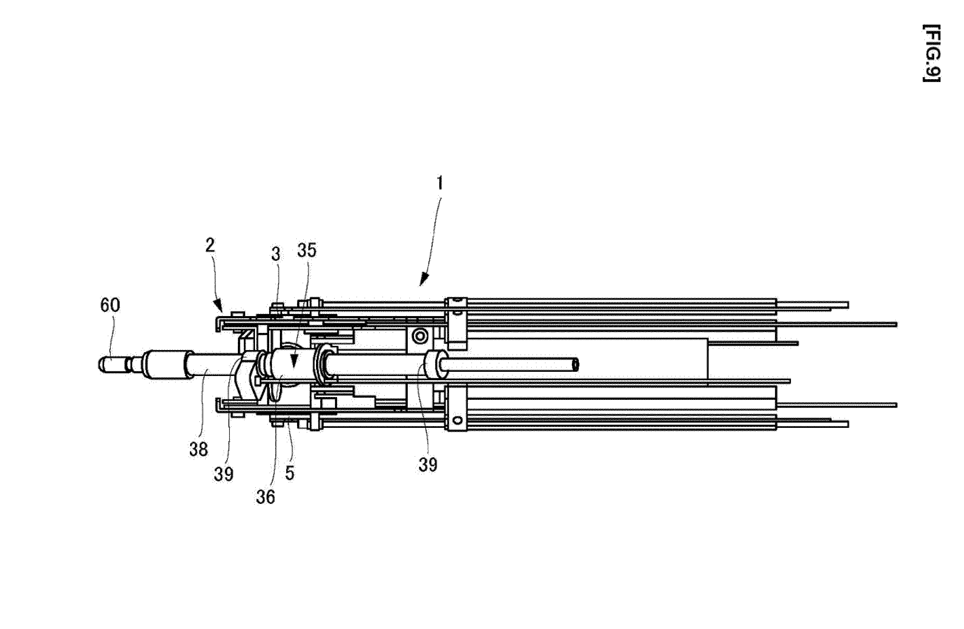

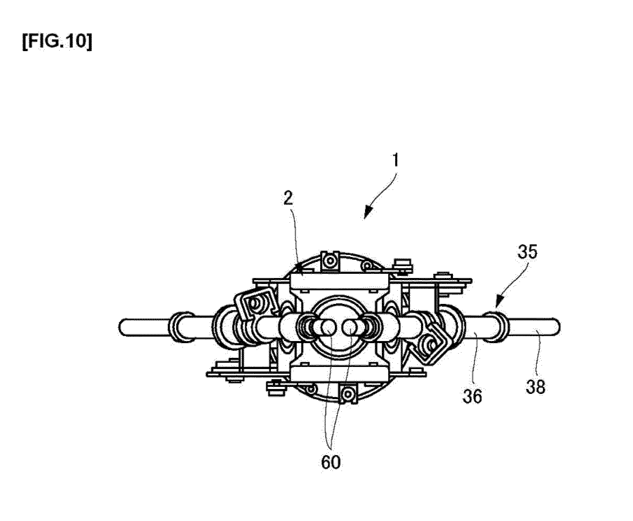

Embodiments of the present invention will now be described through reference to the drawings. FIGS. 1 to 14 show an embodiment of the forceps support device according to the present invention. A forceps support device 1 according to this embodiment is effective at supporting forceps used in endoscopic treatment, and particularly in minimally invasive endoscopic treatment performed on a disorder in the digestive tract or the like inside the abdominal cavity, for example. As shown in FIGS. 1 to 5 and FIGS. 6 to 9, this device includes a frame 2, a pair of forceps support portions 11 provided at both ends of the frame 2, an endoscope support portion 45 provided inside the frame 2, forceps support means 20 provided to each of the forceps support portions 11 to support the forceps 60 pivotably in the horizontal direction and the vertical direction, endoscope support means 46 provided to the endoscope support portion 45 for supporting an endoscope (not shown) at the endoscope support portion 45 so as to allow forward and backward movement, and drive means for pivoting forceps 60 via the support means 20 in the horizontal direction and the vertical direction and moving the endoscope back and forth via the endoscope support means 46. With the present invention, the shape of the endoscope support portion 45 is defined so that an existing endoscope can be supported without any modification.

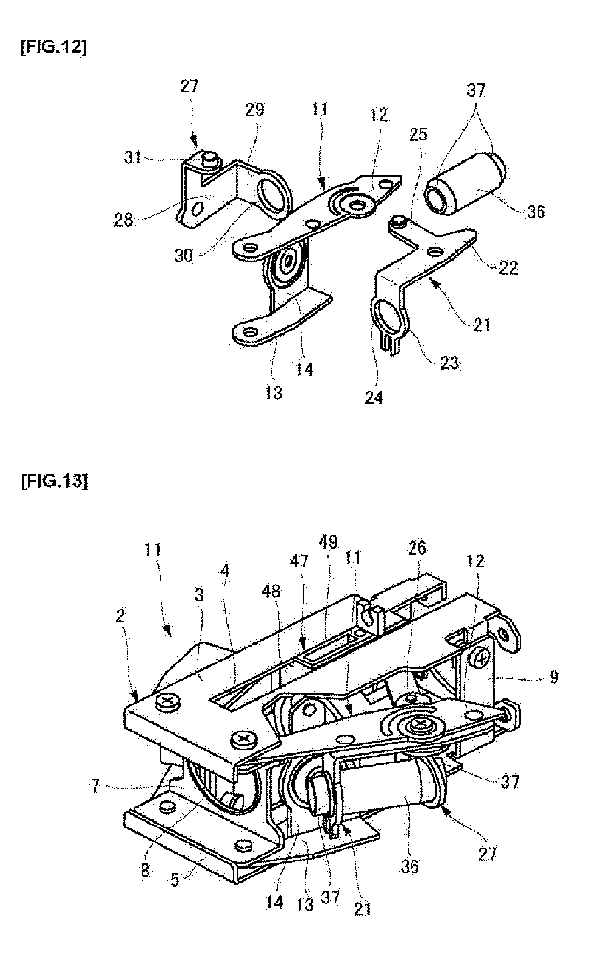

As shown in FIG. 13, the frame 2 is substantially box shaped and is constituted by an upper plate 3 and a lower plate 5 that are quadrangular in shape and are provided parallel to each other and separated in the up and down direction, a front plate 7 that is quadrangular in shape and is linked by screws at one end in the lengthwise direction of the upper plate 3 and the lower plate 5, and a pair of band-shaped side plates that are linked by screws at the other end in the lengthwise direction of the upper plate 3 and the lower plate 5. The pair of forceps support portions 11 are provided on either side of this frame 2.

In this embodiment, each side plate 9 is formed integrally with the upper plate 3 and the lower plate 5, and the end of each side plate 9 is linked by a screw to the lower plate 5 and the upper plate 3, but the constitution may instead be such that each side plate 9 is formed separately from the upper plate 3 and the lower plate 5, and both ends of each side plate 9 are linked via screws between the rear ends of the upper plate 3 and the lower plate 5.

As shown in FIGS. 12 and 13, the forceps support portions 11 each consist of a band-shaped top plate 12, a band-shaped bottom plate 13 provided parallel to the top plate 12 and at a distance below it, and a band-shaped side plate 14 that links the top plate 12 and the bottom plate 13 in the up and down direction. One end in the lengthwise direction of the top plate 12 is linked by a screw to one end in the lengthwise direction of the upper plate 3 of the frame 2, and one end in the lengthwise direction of the bottom plate 13 is linked by a screw to one end in the lengthwise direction of the lower plate 5 of the frame 2.

Each of the forceps support portions 11 is such that the top plate 12 and the bottom plate 13 are linked to the upper plate 3 and the lower plate 5 of the frame 2 so that they spread outward in a V shape with respect to the two side faces of the frame 2, and the opening angle of this V shape (such as an angle between the forceps support portions 11 of about 30 to 60 degrees, and preferably about 40 degrees) with respect to the frame 2 can be adjusted by operating a suitable drive means, and as a result the angle of the forceps 60 supported by the forceps support portions 11 via the forceps support means 20 (discussed below) can be adjusted with respect to the frame 2. Therefore, during insertion into the body, the forceps support portions 11 are closed so as to be in contact with the frame 2, which reduces the cross sectional area of the device as a whole and has less impact on the body, whereas at the affected treatment site the V-shaped opening angle of the forceps support portions 11 can be adjusted as needed so that the operating range of the forceps can be increased.

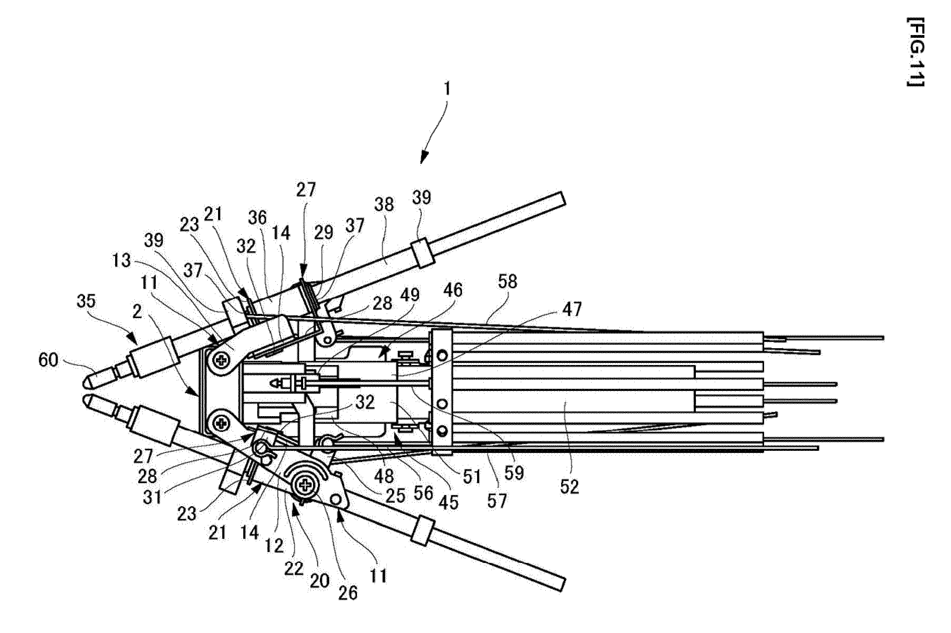

As shown in FIGS. 12 to 14, the forceps support means 20 each consist of a first arm 21 that is rotatably linked via a first pin 26 to the other end in the lengthwise direction of the top plate 12 of the forceps support portion 11 and can pivot horizontally (yaw) around the first pin 26, a second arm 27 that is rotatably linked via a second pin 32 to the side plate 14 of the forceps support portion 11 and can pivot vertically (pitch) around the second pin 32 (see FIG. 11), and a forceps support member 35 that spans the first arm 21 and the second arm 27 horizontally and can pivot horizontally and vertically by following the first arm 21 and the second arm 27. Since there is a specific distance between the first and second pins, adequate force is transmitted to the operating axis in the yaw and pitch directions, while at the same time the overall size of the device can be kept small.

As shown in FIG. 12, the first arm 21 is formed in an L shape by bending a band-shaped plate in its middle in the lengthwise direction, and includes an attachment portion 22 that is rotatably linked via the first pin 26 to the lower face side of the top plate 12 of the forceps support portion 11, a forceps attachment portion 23 that hangs down from one end in the lengthwise direction of the attachment portion 22 and has an insertion hole 24 in its middle for inserting the forceps support member 35, and a lever 25 that protrudes horizontally from one end in the width direction of the attachment portion 22. As shown in FIG. 13, the constitution is such that everything can pivot horizontally around the first pin 26.

As shown in FIG. 12, the second arm 27 is formed in an L shape by bending a band-shaped plate in its middle in the lengthwise direction, and includes an attachment portion 28 that is rotatably linked via the second pin 32 (see FIG. 11) to the inner face side of the side plate 14 of the forceps support portion 11, a forceps attachment portion 29 that protrudes horizontally from one end in the lengthwise direction of the attachment portion 28 and has an insertion hole 30 in its middle for inserting the forceps support member 35, and a lever 31 that protrudes upward in an L shape from the other end in the lengthwise direction of the attachment portion 28. As shown in FIG. 13, the constitution is such that everything can pivot vertically around the second pin 32.

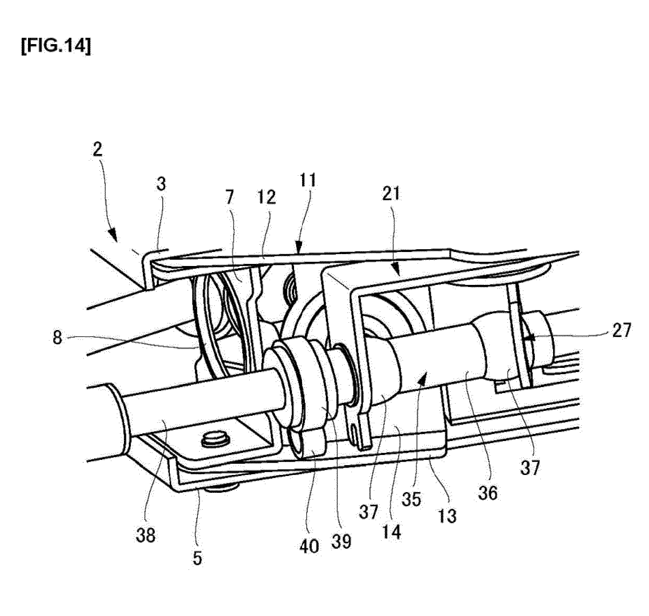

As shown in FIG. 14, the forceps support member 35 includes an outer tube 36 that spans the forceps attachment portion 23 of the first arm 21 1 and the forceps attachment portion 29 of the second arm 27, and an inner tube 38 that is rotatably and slidably inserted on the inner peripheral side of the outer tube 36. The forceps 60 are attached to the inner peripheral side of the inner tube 38.

The forceps support member 35 is such that both ends of the outer tube 36 are inserted between the insertion hole 24 of the forceps attachment portion 23 of the first arm 21 and the insertion hole 30 of the forceps attachment portion 29 of the second arm 27, and bearing portions 37 at both ends are engaged with the inner peripheral face of the insertion hole 24 of the first arm 21 and the inner peripheral face of the insertion hole 30 of the second arm 27, thereby spanning the forceps attachment portion 23 of the first arm 21 and the forceps attachment portion 29 of the second arm 27.

Each of the bearing portions 37 has a shape (such as a spherical surface) that rotatably engages both ends of the outer tube 36 to the inner peripheral face of the insertion hole 24 of the first arm 21 and to the inner peripheral face of the insertion hole 30 of the second arm 27. When the forceps support member 35 is pivoted horizontally and integrally with the first arm 21, this bearing portion 37 allows the portion of the outer tube 36 of the forceps support member 35 that comes into contact with the inner peripheral face of the insertion hole 30 of the second arm 27 to rotate. Also, when the forceps support member 35 is pivoted vertically and integrally with the second arm 27, the portion of the outer tube 36 of the forceps support member 35 that comes into contact with the inner peripheral face of the insertion hole 24 of the first arm 21 rotates via the bearing portion 37. Consequently, when the forceps support members 35 are smoothly pivoted in the horizontal and vertical directions integrally with the first arm 21 and the second arm 27 by a drive means (discussed below), the forceps 60 supported by the forceps support members 35 can be smoothly pivoted in the horizontal and vertical directions.

As shown in FIG. 11, tubular stoppers 39 are provided integrally to both ends of the inner tube 38, and the inner tube 38 is designed to be able to slide within the range over which the end faces in the lengthwise direction of the outer tube 36 hit the end faces of these stopper 39. When the inner tube 38 is slid on the inner peripheral side of the outer tube 36, this causes the forceps 60 to slide integrally with the inner tube 38 in the same direction. One end of a third wire 54 of a drive means 51 (discussed below) is linked to the outer peripheral face of one of the stoppers 39.

The interior of the frame 2 is constituted by the endoscope support portion 45 that supports an endoscope, and the endoscope is provided to this endoscope support portion 45 via the endoscope support means 46 so as to be able to move back and forth in the lengthwise direction of the frame 2.

As shown in FIG. 11, the endoscope support means 46 includes a sliding member 47 that can slide on the endoscope support portion 45, and a tubular member 52 that is fitted on the inside of the sliding member 47. An endoscope (not shown) is fitted to the inner peripheral side of the tubular member 52.

The sliding member 47 is constituted by a substantially tubular front member 48 and a substantially tubular rear member 51 whose distal end is fitted to the rear end of the front member 48. The distal end of the endoscope is fitted via the tubular member 52 to the inner peripheral side of the front member 48 and the rear member 51.

Protrusions 49 that protrude upward and downward are integrally provided to the upper and lower ends of the front member 48 of the sliding member 47, the upper protrusion 49 is engaged in a sliding groove 4 provided in the center part of the upper plate 3 of the frame 2, and the lower protrusion 49 is engaged in a sliding groove 4 provided in the center part of the lower plate 5 of the frame 2, the result being that the sliding member 47 is supported so as to be able to move back and forth in the lengthwise direction of the frame 2.

The endoscope can be moved back and forth in the lengthwise direction of the frame 2 via the sliding member 47 and the tubular member 52, which allows the lens of the endoscope to be moved back and forth, and allows the position of the lens in the forward and backward direction to be adjusted.

An opening 8, through which is inserted the lens of the endoscope supported by the sliding member 47 via the tubular member 52, is provided in the center part of the front plate 7 of the frame 2, and this opening 8 prevents the lens from interfering with the front plate of the frame 2 when the endoscope is moved back and forth.

One end of a fourth wire 59 of a drive means 55 (discussed below) is linked to the upper protrusion 49 of the front member 48 of the sliding member 47, and the sliding member 47 moves forward and backward via the fourth wire 59.

As shown in FIG. 11, the drive means includes a first wire 56 that is linked at one end to the lever 25 of the first arm 21 of each of the forceps support means 20 and is able to move back and forth in the lengthwise direction of the frame 2, a first actuator (not shown) that drives the first wire 56, a second wire 57 that is linked at one end to the lever 31 of the second arm 27 and is able to move back and forth in the lengthwise direction of the frame 2, a second actuator (not shown) that drives the second wire 57, a third wire 58 that is linked at one end to the inner tube 38 of the forceps support member 35 and is able to move back and forth in the lengthwise direction of the frame 2, a third actuator (not shown) that drives the third wire 58, a fourth wire 59 that is linked at one end to the sliding member 47 and is able to move back and forth in the lengthwise direction of the frame 2, and a fourth actuator (not shown) that drives the fourth wire 59.

The first actuator of the drive means is actuated to pivot the first arm 21 of the forceps support means 20 horizontally via the first wire 56, the result being that the forceps support member 35 spanning the first arm 21 1 and the second arm 27 follows the first arm 21 and pivots in the same direction, the forceps 60 supported by the forceps support member 35 pivot in the same direction (horizontally), and the pivot position of the forceps 60 in the horizontal direction is adjusted.

Also, the second actuator is actuated to pivot the second arm 27 of the forceps support means 20 vertically via the second wire 57, the result being that the forceps support member 35 spanning the first arm 21 and the second arm 27 follows the second arm 27 and pivots in the same direction, the forceps 60 supported by the forceps support member 35 pivot in the same direction (vertically), and the pivot position of the forceps 60 in the vertical direction is adjusted.

Further, the third actuator is actuated to slide the inner tube 38 of the forceps support member 35 via the third wire 58, the result being that the forceps 60 supported on the inner peripheral side of the inner tube 38 follow the inner tube 38 and slide in the same direction, and the position of the distal end of the forceps 60 in the forward and backward direction is adjusted.

Further, the fourth actuator is actuated to move the sliding member 47 forward or backward via the fourth wire 59, the result being that the lens of the endoscope supported by the sliding member 47 can be moved in the same direction, and the position of the lens in the forward and backward direction can be adjusted.

As shown in FIGS. 1 to 5, for example, the pair of forceps 60 are position in the horizontal and vertical directions so as to be parallel to each other, in this state are inserted into the interior of the body through the nose, mouth, etc., and are positioned at a position corresponding to the disorder in the digestive tract or the like.

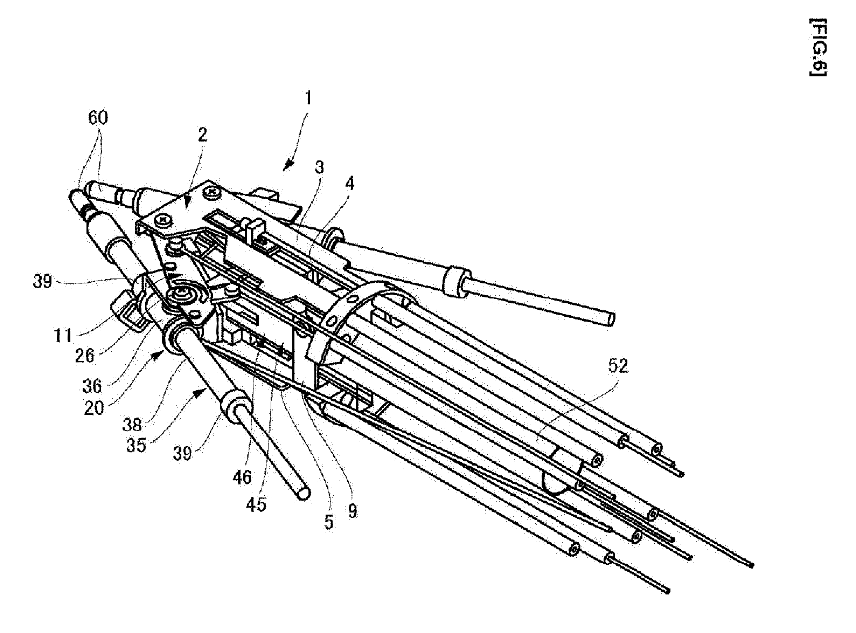

As shown in FIGS. 6 to 10, the pair of forceps 60 are pivoted in the horizontal and vertical directions from the state shown in FIGS. 1 to 5 to a state in which the distal ends of the forceps 60 are close together, and further rotation from this state allows various kinds of treatment to be performed on the disorder between the distal ends of the forceps 60.

With the forceps support device 1 in this embodiment constituted as above, the two forceps support portions 11 are provided to both sides of the frame 2, the forceps 60 are supported by the forceps support portions 11 via the forceps support means 20, and the first arms 21 of the forceps support means 20 are pivoted horizontally by manipulation of the first wire 56 of the drive means, the result being that the forceps 60 follow the first arms 21 and pivot in the same direction via the forceps support members 35, and the second arms 27 are pivoted horizontally by manipulation of the second wire 57 of the drive means, the result being that the forceps 60 follow the second arms 27 and pivot in the same direction via the forceps support members 35. Because of this constitution, there is no response lag, the forceps 60 can be positioned accurately at the desired position in the horizontal and vertical directions, and fine tuning of the resulting position in the horizontal and vertical directions can be performed very precisely.

Furthermore, since the forceps 60 can each be pivoted independently in the horizontal and vertical directions by the respective forceps support means 20, the angles of the two forceps 60 can be adjusted as desired, allowing them to better accommodate various kinds of disorder in the digestive tract or the like, and allowing the range of applications to be expanded.

Furthermore, since the forceps support member 35 is constituted so that the inner tube 38 on the inner peripheral side of the outer tube 36 can be slid by manipulation of the third wire 58, fine tuning in the forward and backward direction of the forceps 60 supported on the inner peripheral side of the inner tube 38 can also be performed more precisely.

Furthermore, since the lens of the endoscope can be moved in the forward and backward direction by sliding the sliding member 47 by manipulation of the fourth wire 59, adjustment of the position of the endoscope in the forward and backward direction can also be performed more precisely.

It should go without saying that the first to fourth wires 56, 57, 58, and 59 may be manipulated manually instead of using drive means, and these wires may be substituted with rods, or the wires or rods can be substituted with some drive means other than that discussed above.

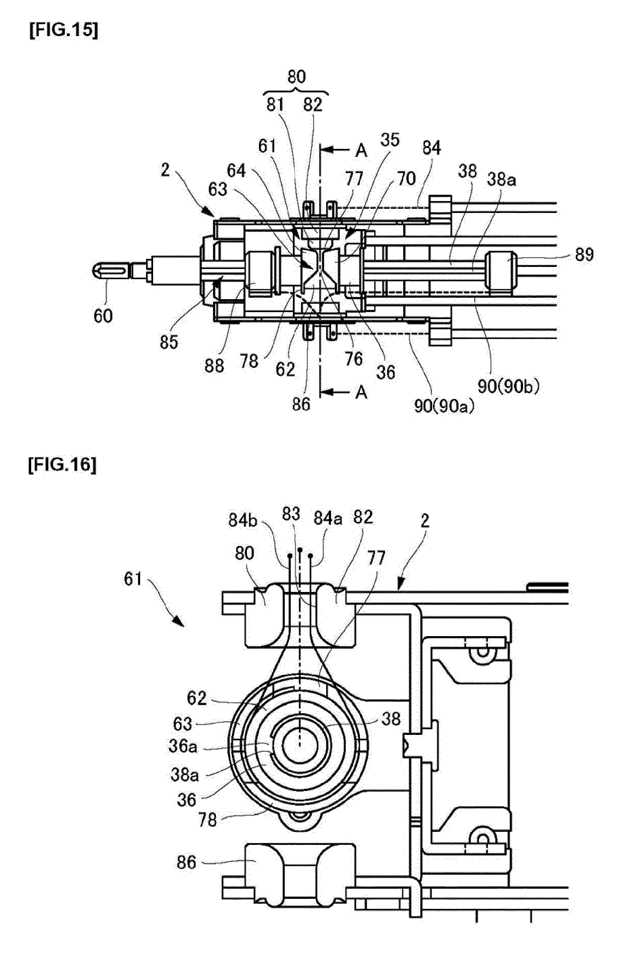

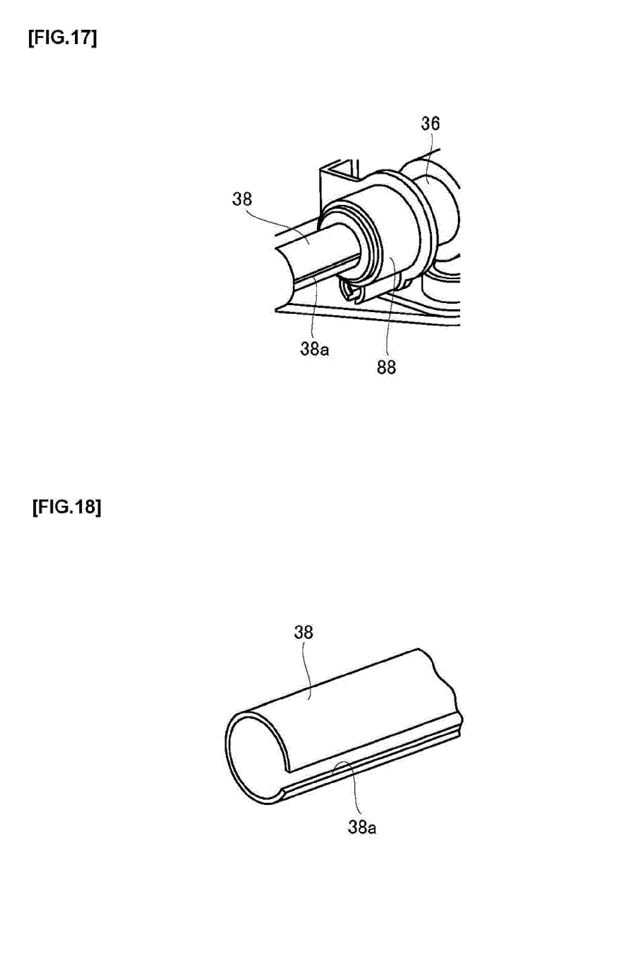

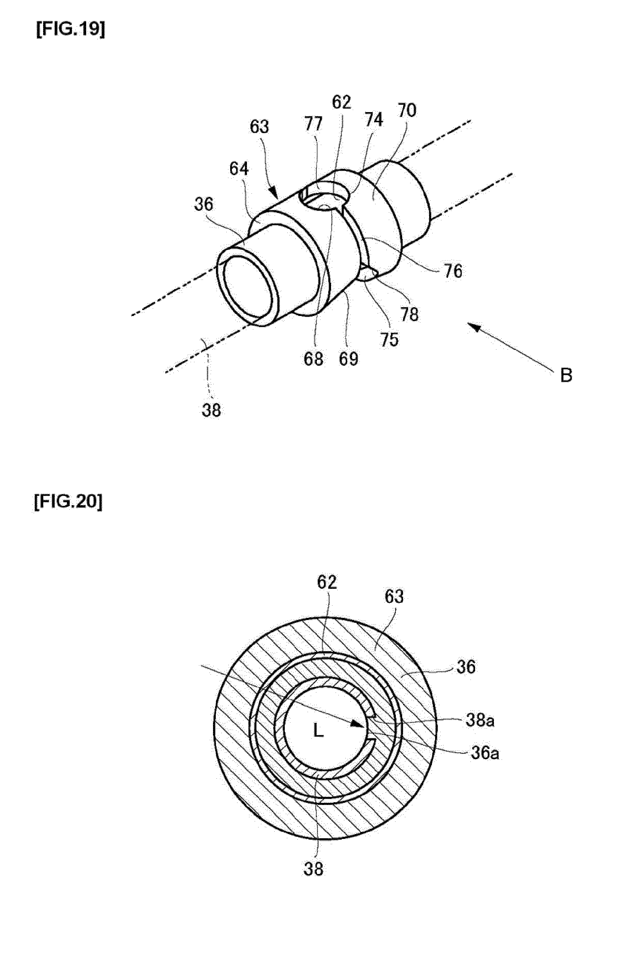

FIGS. 15 to 21 show another embodiment of the forceps support device according to the present invention. As shown in FIG. 15, the forceps support device 1 in this embodiment includes a rotation mechanism 61 that supports the forceps 60 so that each can rotate on the frame 2 and can be positioned at the desired position in the rotation direction, and a sliding mechanism 85 that supports the forceps 60 so that each can move back and forth on the frame 2 and can be positioned at the desired position in the forward and backward direction. The rest of the constitution is the same as that in the above embodiment.

As shown in FIGS. 15, 16 19, and 21, the rotation mechanism 61 includes a tubular wire attachment portion 62 that is larger in diameter than the outer tube 36 and is provided integrally in the center part of the forceps support member 35 in the lengthwise direction on the outer peripheral side of the outer tube 36, a tubular wire guide 63 that is larger in diameter than the wire attachment portion 62 and is provided so as to be capable of relative rotation on the outer peripheral side of the wire attachment portion 62, a rotation restricting pin 80 that is fixed to the upper part of the frame 2 and whose distal end (lower end) is engaged with the top of the wire guide 63, and a pair of fifth wires 84 (84a and 84b) that rotate around the wire attachment portion 62 in mutually opposite directions, are linked at one end to the top of the wire attachment portion 62, at the other end pass through the center part of the rotation restricting pin 80, coming out at the upper part of the frame 2, and are taken out to the right in FIG. 15 along the upper part of the frame 2.

The wire guide 63 is made up of a tubular first guide 64 that is provided so as to be capable of relative rotation on the outer peripheral side of the portion of the wire attachment portion 62 on the front side (the portion on the left side in FIG. 15), and a tubular second guide 70 that is provided so as to be capable of relative rotation on the outer peripheral side of the portion of the wire attachment portion 62 on the rear side the (portion on the right in FIG. 15).

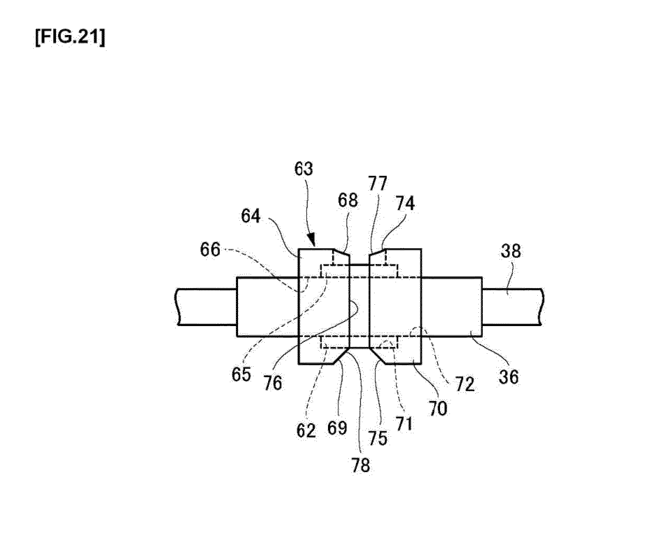

As shown in FIG. 21, the first guide 64 has an inner peripheral face that is formed in two levels consisting of a large-diameter part 65 and a small-diameter part 66. The outer tube 36 is inserted on the inner peripheral side of the small-diameter part 66, the portion of the wire attachment portion 62 on the front side is inserted on the inner peripheral side of the large-diameter part 65, and the end face of the wire attachment portion 62 on the front side is brought into contact with the interface between the small-diameter part 66 and the large-diameter part 65, the result being attachment that allows relative rotation on the outer peripheral side of the portion of the wire attachment portion 62 on the front side.

As shown in FIG. 21, the second guide 70 is similar to the first guide 64 in that its inner peripheral face is formed in two levels consisting of a large-diameter part 71 and a small-diameter part 72. The outer tube 36 is inserted on the inner peripheral side of the small-diameter part 72, the portion of the wire attachment portion 62 on the rear side is inserted on the inner peripheral side of the large-diameter part 71, and the end face of the wire attachment portion 62 on the rear side is brought into contact with the interface between the small-diameter part 72 and the large-diameter part 71, the result being attachment that allows relative rotation on the outer peripheral side of the portion of the wire attachment portion 62 on the front side.

As shown in FIGS. 15, 19, and 21, because the first guide 64 and the second guide 70 are attached to the outer peripheral side of the small-diameter part 72, a guide groove 76 of a specific width that goes all the way around the small-diameter part 72 is provided between the two guides 64 and 70, and this guide groove 76 guides the pair of fifth wires 84 in a state of being wound around the wire attachment portion 62.

The first guide 64 and the second guide 70 are supported so as to be capable of relative rotation at a specific location on the outer peripheral side of the wire attachment portion 62 by, for example, an annular groove (not shown) being provided in the portion of the outer peripheral face of the outer tube 36 that is adjacent to the end face on the front side of the first guide 64 and the end face on the rear side of the second guide 70, respectively, and a then stop wheel (not shown) being fitted into each of these grooves.

As shown in FIGS. 15, 19, and 21, at the upper part of the guide groove 76, a substantially circular engagement hole 77 is formed by substantially semicircular upper cut-outs 68 and 74 provided to the face of the first guide 64 that is opposite the second guide 70 and to the face of the second guide 70 that is opposite the first guide 64. The distal end of the rotation restricting pin 80 is engaged in this engagement hole 77, which allows the forceps 60 to be rotated via the wire attachment portion 62, the outer tube 36 and the inner tube 38 on the inner peripheral face side of the first guide 64 and the second guide 70, while rotation of the first guide 64 and the second guide 70 is restricted (the first guide 64 and the second guide 70 are fixed on the frame 2 side).

As shown in FIGS. 15, 19, and 21, at the lower part of the guide groove 76, a substantially hexagonal guide hole 78 is formed by substantially trapezoidal lower cut-outs 69 and 75 provided to the face of the first guide 64 that is opposite the second guide 70 and to the face of the second guide 70 that is opposite the first guide 64, respectively. This guide hole 78 prevents tangling of the pair of fifth wires 84 that are guided by the guide groove 76.

As shown in FIG. 16, the fifth wire 84a is linked at one end to the top of the wire attachment portion 62, and the other end is wound counter-clockwise around the wire attachment portion 62 and pulled out upward, is taken through the center part of the rotation restricting pin 80 to the upper part of the frame 2, and is taken out to the right in FIG. 15 along the upper part of the frame 2. The other fifth wire 84b is linked at one end to the top of the wire attachment portion 62, and the other end is wound clockwise around the wire attachment portion 62 and pulled out upward, is taken through the center part of the rotation restricting pin 80 to the upper part of the frame 2, and is taken out to the right in FIG. 15 along the upper part of the frame 2.

As shown in FIGS. 15 and 16, the rotation restricting pin 80 includes a dowel-shaped engagement portion 81 whose distal end (lower end) is formed as a curved surface, and a disk-shaped proximal portion 82 that is provided integrally with the upper end of the engagement portion 81 and is fixed to the first arm 21 of the frame 2. A through-hole 83 is provided that passes through the center of the engagement portion 81 and the proximal portion 82 in the axial direction, and the pair of fifth wires 84 pass through this through-hole 83 from below to above. Only the proximal portion 82 of the rotation restricting pin 80 is shown in FIG. 16.

As shown in FIG. 15, with the rotation restricting pin 80, the distal end of the engagement portion 81 is engaged in the engagement hole 77 formed between the upper cut-out 68 of the first guide 64 and the upper cut-out 74 of the second guide 70 of the wire guide 63, and this restricts the rotation of the first guide 64 and the second guide 70 of the wire guide 63, and allows the forceps 60 to rotate on the inner peripheral face side of the first guide 64 and the second guide 70 via the wire attachment portion 62, the outer tube 36, and the inner tube 38.

The one of the fifth wires 84a is pulled to the right in FIG. 15, and as a result the wire attachment portion 62 rotates counter-clockwise on the inner peripheral side of the wire guide 63, while the outer tube 36, the inner tube 38, and the forceps 60 rotate in the same direction and integrally with the wire attachment portion 62. The other fifth wire 84a is pushed to the left in FIG. 15, and as a result the wire attachment portion 62 rotates clockwise on the inner peripheral side of the wire guide 63, and the outer tube 36, the inner tube 38, and the forceps 60 rotate in the same direction and integrally with the wire attachment portion 62.

Furthermore, one of the other fifth wires 84b is pulled to the right in FIG. 15, the wire attachment portion 62 rotates clockwise on the inner peripheral side of the wire guide 63, and the outer tube 36, the inner tube 38, and the forceps 60 rotate in the same direction and integrally with the wire attachment portion 62. The other is pushed to the left in FIG. 15, and as a result the wire attachment portion 62 rotates counter-clockwise on the inner peripheral side of the wire guide 63, and the outer tube 36, the inner tube 38, and the forceps 60 rotate in the same direction and integrally with the wire attachment portion 62.

As shown in FIGS. 15, 17, 18, and 20, the sliding mechanism 85 includes a sliding groove 38a that is provided to part of the inner tube 38 of the forceps support member 35 and has a specific width extending over the entire length of the inner tube 38 passing between the inner and outer peripheral faces of the inner tube 38, a protrusion 36a that is provided over the entire length of the outer tube 36 and in a state of protruding inward at the portion of the inner peripheral face of the outer tube 36 corresponding to the sliding groove 38a and that is slidably engaged in the sliding groove 38a of the inner tube 38, a first stopper 88 that is provided integrally with the outer peripheral side portion of the inner tube 38 on the front side of the outer tube 36 (the left side in FIG. 15), a second stopper 89 that is provided integrally with the outer peripheral side portion of the inner tube 38 on the rear side of the outer tube 36 (the right side in FIG. 15), and a pair of sixth wires 90 that are linked at one end to the first stopper 88 or the second stopper 89 and are taken out at the other end to the right in FIG. 15. A relative rotation body 88a that is capable of relative rotation with the first stopper 88 and to which the inner tube 38 is fixed is provided on the inside of the first stopper 88. A similar relative rotation body (not shown) is also provided on the inside of the second stopper 89.

One of the sixth wires 90a is linked at one end to the first stopper 88, and the other end is taken out in the up and down direction through a tubular wire support portion 86 fixed to the portion of the bottom plate 13 of the frame 2 under the wire guide 63, to the lower part of the bottom plate 13, and is taken out to the right in FIG. 15 along the lower part of the bottom plate 13. The other sixth wire 90b is linked at one end to the second stopper 89, and the other end is first taken out forward along the upper part of the bottom plate 13 of the frame 2, taken out in the up and down direction through the wire support portion 86 to the lower part of the bottom plate 13, and taken out to the right in FIG. 15 along the lower part of the bottom plate 13.

The sixth wire 90a is pulled to the right in FIG. 15, which pulls the first stopper 88 to the right in FIG. 15, causing the inner tube 38 to move to the right integrally with the first stopper 88, and the forceps 60 provided integrally on the inside of the inner tube 38 to move to the right in FIG. 15. When the other sixth wire 90b is pulled to the right in FIG. 15, this pulls the second stopper 89 to the left in FIG. 15, causing the inner tube 38 to move to the left in FIG. 15 integrally with the second stopper 89, and the forceps 60 provided integrally on the inside of the inner tube 38 to move to the left in FIG. 15.

In this case, the protrusion 36a of the outer tube 36 is engaged in the sliding groove 38a of the inner tube 38, and in this state the inner tube 38 moves in the axial direction relative to the outer tube 36 while co-rotating with the outer tube 36, so the rotation of the forceps 60 via the inner tube 38 is permitted without tangling of the one sixth wire 90a, and the forceps 60 can be moved back and forth in the axial direction.

As shown in FIG. 15, the inner tube 38 and the forceps 60 can move in the forward and backward direction on the inner peripheral side of the outer tube 36 within the range over which the first stopper 88 hits the end face on the front side of the outer tube 36 and the second stopper 89 hits the end face on the front side of the outer tube 36.

With the forceps support device 1 in this embodiment constituted as above, in addition to the same action and effects discussed in the above embodiment, the forceps 60 can be rotated by the rotation mechanism 61 and positioned at the desired position in the rotation direction, and the forceps 60 can be moved in the forward and backward direction by the sliding mechanism 85 and positioned at the desired position in the forward and backward direction, so this device can accommodate various forms of disorder in the digestive tract or the like, and the scope of applicability can be further expanded.

In this embodiment, the fifth wires 84 and the sixth wires 90 may be operated manually, or may be operated by a drive means, or the wires may be substituted with rods.

### Reference Signs List

- 1: forceps support device
- 2: frame
- 3: upper plate
- 4: sliding groove
- 5: lower plate
- 6: sliding groove
- 7: front plate
- 8: opening
- 9: side plate
- 11: forceps support portion
- 12: top plate
- 13: bottom plate
- 14: side plate
- 20: forceps support means
- 21: first arm
- 22: attachment portion
- 23: forceps attachment portion
- 24: insertion hole
- 25: lever
- 26: first pin
- 27: second arm
- 28: attachment portion
- 29: forceps attachment portion
- 30: insertion hole
- 31: lever
- 32: second pin
- 35: forceps support portion
- 36: outer tube
- 36a: protrusion
- 37: bearing portion
- 38: inner tube
- 38a: sliding groove
- 39: stopper
- 45: endoscope support portion
- 46: endoscope support means
- 47: sliding member
- 48: front member
- 49: protrusion
- 51: rear member
- 52: tubular member
- 56: first wire
- 57: second wire
- 58: third wire
- 59: fourth wire
- 60: forceps
- 61: rotation mechanism
- 62: wire attachment portion
- 63: wire guide
- 64: first guide
- 65: large-diameter part
- 66: small-diameter part
- 68: upper cut-out
- 69: lower cut-out
- 70: second guide
- 71: large-diameter part
- 72: small-diameter part
- 74: upper cut-out
- 75: lower cut-out
- 76: guide groove
- 77: engagement hole
- 78: guide hole
- 80: rotation restricting pin
- 81: engagement portion
- 82: proximal portion
- 83: through-hole
- 84: fifth wires
- 84a: one fifth wire
- 84b: other fifth wire
- 85: sliding mechanism
- 86: wire support portion
- 88: first stopper
- 89: second stopper
- 90: sixth wires
- 90a: one sixth wire
- 90b: other sixth wire

## Claims

1. A forceps support device for supporting forceps used for endoscopic treatment, comprising:
a frame;
a pair of forceps support portions provided at both ends of the frame;
forceps support means provided to each of the forceps support portions to support the forceps pivotably in the horizontal direction and the vertical direction; and
drive means for pivoting the forceps support means in the horizontal direction and the vertical direction.

2. The forceps support device according to claim 1,
wherein the forceps support means comprises:
a first arm joined to each of the forceps support portion pivotably in the horizontal direction;
a second arm joined to each of the forceps support portion pivotably in the vertical direction; and
a forceps support member that spans the first arm and the second arm, is pivotable in the horizontal direction while following the first arm, and pivotable in the vertical direction while following the second arm, and
wherein the forceps are supported by the forceps support members.

3. The forceps support device according to claim 2,
wherein the drive means comprises:
a first wire that is linked at one end to the first arm and can move back and forth in the lengthwise direction of the frame;
a second wire that is linked at one end to the second arm and can move back and forth in the lengthwise direction of the frame; and
an actuator for moving the first wire and the second wire back and forth.

4. The forceps support device according to claim 2, wherein the forceps support members support the forceps rotatably and slidably.

5. The forceps support device according to claim 4,
wherein the forceps support members comprises an outer tube that spans the first arm and the second arm; and
an inner tube that is slidably inserted on the inner peripheral side of the outer tube, and
wherein bearing portions that rotatably engage the outer tube with the first arm and the second arm are provided at both ends of the outer tube.

6. The forceps support device according to any of claims 1 to 5, wherein an endoscope support portion is provided to the frame, and an endoscope is supported by the endoscope support portion, via endoscope support means, so as to be able to move back and forth in the lengthwise direction of the frame.

7. The forceps support device according to claim 6,
wherein the endoscope support means comprises a slide member mounted slidably in the lengthwise direction to the frame, and the endoscope is supported by the slide member.
